# EUROPEAN PATENT SPECIFICATION

(11) **EP 1 370 248 B1**
(45) Date of publication and mention of the grant of the patent: **22.09.2010**
(21) Application number: 02706353.6
(22) Date of filing: 22.02.2002
(51) Int. Cl.: A61K 38/17, A61P 7/02

(54) **COMPOSITIONS FOR PREVENTING PLATELET AGGEGATION**
ZUSAMMENSETZUNGEN ZUR VERHINDERUNG DER THROMBOZYTENAGGREGATION
COMPOSITIONS DESTINES A PREVENIR L'AGREGATION PLAQUETTAIRE

(30) Priority: 22.02.2001 US 270759 P
(43) Date of publication of application: 17.12.2003
(73) Proprietor: Philadelphia Health and Education Corporation, Philadelphia, PA 19102 (US); Symbiotec GmbH, 66123 Saarbrücken (DE)
(72) Inventor: CLASS, Reiner, 66740 Saarlouis (DE); SOSLAU, Gerald, Feasterville, PA 19053 (US); ZEPPEZAUER, Michael, 66133 Saarbrücken (DE)
(74) Representative: Pätzold, Herbert
(86) International application number: PCT/US2002/005157
(87) International publication number: WO 2002/067907

(56) References cited:
- WO-A-01/10901
- WO-A-99/37318
- US-A- 5 126 140
- US-A- 5 607 691
- US-A- 5 616 311
- KLOCZKO J; CZACZKOWSKA T; WOJTUKIEWICZ M; BIELAWIEC M: "INFLUENCE OF HISTONE-CHONDROITIN SULFATE A COMPLEXES ON BLOOD PLATELET AGGREGATION" FOLIA HAEMATOLOGICA (LEIPZIG), vol. 113, no. 4, 1986, pages 545-549, XP009101194 ISSN: 0323-4347
- KHEIRI S A; FASY T M; BILLETT H H: "Effects of H1 histones and a monoclonal autoantibodies to H1 histones on clot formation in vitro: Possible implications in the antiphospholipid syndrome" THROMBOSIS RESEARCH, vol. 82, no. 1, 1996, pages 43-50, XP002267521 ISSN: 0049-3848

## Description

### BACKGROUND OF THE INVENTION

Despite advances in anti-platelet and anti-thrombotic treatment regimens, cardiovascular diseases remain the leading cause of death in the United states. Clinically employed anti-platelet and anti-thrombotic agents include heparin, aspirin, integrilin, and anti-GP IIb/IIIa antibodies (c7E3 Fab, abciximab, or ReoPro). α-Thrombin, generated at the site of vessel injury, is generally assumed to catalyze the hydrolysis of an N-terminai peptide from the human platelet 7-transmembrane thrombin receptor, protease activated receptor 1 (PAR-1), which initiates a cascade of molecular reactions leading to thrombus formation.

Early studies of the cellular thrombin receptor indicated that more than one species exists in platelets (Greco, N.J. and G.A. Jamieson. 1991. PSEEM 198:792-799; Harmon, J.T. and G.A. Jamieson. 1986. J. Biol. Chem. 261:15928-15933). The first cellular thrombin receptor cloned and sequenced was PAR-1 (Vu, T-K.H. et al. 1991. Cell 64:1057-1068). Human platelets respond to the activation of PAR-1 and a second minor receptor PAR-4 (Kahn, M.L. et al. 1998. Nature 394:690-694) while the recently cloned PAR-3 is either absent on human platelets or present in trace amounts (Ishihara, H. et al. 1997. Nature 386:502-506). Mouse platelets respond to α-thrombin primarily through PAR-3 and secondarily through PAR-4, with no involvement of PAR-1 (Vu, T-K.H. et al. 1991. Cell 64:1057-1068). Another platelet membrane protein, GP Ib, may also function at least in part as a thrombin receptor (Greco, N.J. and G.A. Jamieson. 1991. FSEBM 198:792-799; Harmon, J.T. and G.A. Jamieson. 1986. J. Biol. Chem. 261:15928-15933; Clemetson, K.J. 1995. Thromb. Haemost. 74:111-116; Greco, N.J. et al. 1996. Biochemistry 35:915).

The physiologic functions of the three purported platelet thrombin receptors (PAR-1, PAR-4 and GP Ib) have not yet been clearly defined. A functional role for PAR-1 in α-thrombin-induced platelet aggregation has been shown in vitro (Vu, T-K.H. et al. 1991. Cell 64:1057-1068) . Human platelets have been shown to respond to γ-thrombin, an autoproteolytic product of α-thrombin, through activation of PAR-4. However, the interactions of anti-thrombotic and anti-platelet therapies with the GP Ib and/or PAR-4 receptor have not been described.

Histones are nuclear proteins involved in DNA packaging, transcription and replication (Isenberg, I. 1979. Ann. Rev. Biochem. 48:159-1191). They are naturally occurring integral components of functional cell membranes (Watson, K. et al. 1994. Biochem. Soc. Trans. 22:199S; Watson, K. et al. 1995. Biochem. Pharmacol. 50:299-309). In the past few years, histones have been found to have many more functions outside of the nucleus and even outside the cell. For example, It has been shown that if a cell undergoes a malignant transformation to a cancer cell, the molecular makeup of the cell is disturbed resulting in a higher percentage of histone molecules in the cell membrane (Class, R. et al. 1996. Am. J. Clin. Oncol. 19:522-531). Histones share homology with classic membrane proteins in that they have a hydrophobic core and hydrophilic C- and N-terminal tails allowing them to insert into the lipid bilayer system. The core domain of histones are composted of cylinder-shaped sequences of α-helices and β-sheets called the histone fold motif. This motif is used in protein oligomerization and channel formation (Arents, et al. 1995. Proc. Natl. Acad. Sci. USA 92:11170-11174).

If normal orientations are changed, histone molecules have the potential to form higher-order complexes with other histones, ultimately resulting in the formation of a potentially lethal channel in the membrane. Such channel formation has been demonstrated in Friend erythroleukemia and Jurkat and ascites tumor cells (Gamberrucci et al. 1998. Biochem. J. 331:623-630). Histone H1 has been shown to bind strongly to artificial cell membranes containing anionic phospholipids (Koiv, A. et al. 1995. Biochemistry 34:8018-8027; Goldberg, E.M. et al. 1998. Eur. J. Biochem. 258:722-728; Subramanian, M. et al. 1998. Biochemistry 3'7:1394-1402). Similar results have been reported in patients with systemic lupus erythematosus, an autoimmune disease involving histone H1 (Pereira et al. 1994. Clin. Exp. Immunol. 97:175-180) . The sub-critical amount of histone molecules in the membrane of cancer cells can be induced to turn on oligomerization and lethal charnel formation by the addition of extracellular histone. H1. Cell lysis, therefore, appears to be critically controlled by the amount of extracelluarly added free histone H1.

The family of histone proteins consists of five members including the core histones H2A, H2B, H3 and H4 and the linker histone H1. Histone H1 has a low immunogenicity and antigenicity, making generation of neutralizing antibodies in patients injected with histone H1 to be an unlikely event. Histone H1 can be used to cross the blood-brain barrier and has been used to treat brain metastases (Partridge et al. 1989. J. Pharmacol. Exp. Ther. 251:821-826).

It has now been found that histones such as H1 have the ability to specifically block γ-thrombin-induced platelet aggregation.

### SUMMARY OF THE INVENTION

An object of the present invention is to provide an agent for presenting platelet aggregation.

Another object of the present invention is to provide an agent for treating cardiovascular disorders related to thrombotic events.

### DERAILED DESCRIPTION OF THE INVENTION

Clinical data has demonstrated that patients taking anti-thrombosis drugs still suffer from relapses, thus indicating that another pathway is active that can induce platelet aggregation in blood vessels. Experiments performed *in vitro* suggest that the γ-thrombin pathway may still be active in patients undergoing anti-platelet or anti-thrombotic therapy, and may be responsible for at least part of the thrombotic response. It has now been found that a composition comprising a histone compound has the ability to specifically and effectively block γ-thrombin-induced platelet aggregation without interfering with platelet aggregation mediated by alpha-thrombin.

The present invention relates to the use of a histone compound and a pharmaceutically acceptable vehicle in the manufacture of an agent for preventing platelet aggregation treatment of cardiovascular disease related to thrombotic events. Compositions of the present invention are comprised of at least one histone compound administered in a pharmaceutically acceptable vehicle directly to an animal, including humans. The histone compound may comprise a H1, H2A, H2B, H3 or H4 histone. In a preferred embodiment the histone compound comprises a H1 histone. The H1 histone may be a H1 subtype such as, H1.0, H1.1, H1.2, H1.3, H1.4, or H1.5. In a preferred embodiment the histone is a human histone. In another preferred embodiment, the histone compound is recombinantly produced. In a preferred embodiment, the pharmaceutically acceptable vehicle comprises isotonic saline. The present invention also provides the use of a histone compound and a pharmaceutically acceptable vehicle in the manufacture of an agent for treating cardiovascular disease reflated to thrombotic events.

The histone compound for an anti platelet agent and an anti thrombotic agent comprising at least one histone.

Studies were performed to examine the role of a composition comprising histone H1 as an anti-thrombotic agent. Platelet aggregation assays were performed in an aggregometer that monitors increasing light transmission as larger platelet aggregates form in solution. γ-Thrombin rapidly induced platelet aggregation in the presence of cofactors. Addition of 5 µl of a composition comprising histone H1 (from a 9.5 µM stock solution) significantly reduced the aggregation of platelets within the observation time period. Administration of a larger dose, 25 µl histone H1 (from a 9.5 µM stock solution) completely abolished platelet aggregation.

Additional experiments were performed with a γ-thrombin mimetic agent, TRAP-4 (Thrombin Receptor Agonist Peptide-4) instead of γ-thrombin itself. TRAP-4 produced a lower level platelet aggregation response. However, addition of a composition comprising histone H1 significantly reduced the level of platelet aggregation induced by TRAP-4.

The effects of a composition comprising histone H1 to inhibit γ-thrombin-induced platelet aggregation were dose-dependent. When doses of 0.95, 4.75 and 9.5 µM histone H1 were added to platelet cells *in vitro*, aggregation was inhibited. a dose of only 0.95 µM histone H1 caused a greater than 95% inhibition of platelet aggregation.

Experiments were also performed to examine the specificity of the interaction of histone H1. When histone H1 was added to α-thrombin-induced platelet preparations, there was no significant effect on α-thrombin-induced platelet aggregation. These results indicate that the interaction of histone H1 is specific for the γ-thrombin pathway.

Studies with other histone compounds such as H2A, H2B, H3, and H4 are indicative of these, histone compounds having similar activity to histone H1. Further the histone H1 and histone H1 subtypes such as, H1.0, H1.1, H1.2, H1.3, H1.4 and H1.5, were both successful in preventing platelet aggregation. Compositions of the present invention comprise a histone compound formulated in a pharmaceutically acceptable vehicle for ingestion or injection as a solution or as a pill form for oral administration. In the context of the present invention by histone compound, it is meant to be inclusive of, but is not limited to the H1, H2A, H2B, H3 and H4 histones, as well as analogs of histones including but not be limited to histone subtypes, histone fragments, recombinantly produced histones, truncated histone, histones with conservative amino acid substitutions, modifications to histones that do not alter the primary and/or secondary structure of the histone (e.g., glycosylation, phosphorylation, methylation, pegylation), D-amino acid-containing histones, and histones fused or linked to other agents. For example, for the H1 histone, useful subtypes include, but are not limited to H1.0, H1.1, H1.2, H1.3, H1.4 and H1.5. Compositions in solution can be injected intravenously, intramuscularly, subcutaneously, intraperitoneally, or instilled directly into the eye or muscosal areas.

Cardiovascular disorders in patients relating to thrombotic events may be treated by administering an effective amount of a histone compound to the patient. Further, compositions of the present invention comprising a histone compound can be given prophylactically to patients at high risk of thrombotic episodes. For example, a composition of the present invention can be administered to a patient following surgery *in lieu* of standard treatments such as heparin. One of skill can administer the compositions of the present invention in accordance with well known techniques in the art. Further, one of skill can routinely select an effective amount of a histone compound to be administered based upon the experimental data provided herein. Extrapolation from effective amount in cells to doses to be administered *in vivo* is performed routine by those skilled in the art.

Histone compounds can also be used to coat implantable devices such as stents or valves, or to coat device inserted in the body of a patient for either short or prolonged periods such: as catheters. In the context of the present invention an implantable device would include devices that are placed inside the blood vessels or the heart for any amount of time, either short-term or long-term. To prepare the coating, the histone compound is formulated in a physiologically acceptable matrix. In the context of the present invention a physiologically acceptable matrix is one that is not recognized as foreign to the immune system and is biocompatible (a substance that does not produce any irritation, immunogenic, or inflammatory response).

The following non-limiting examples are provided to further illustrate the present invention.

### EXAMPLES

### Example 1: Platelet Aggregation Assay

Blood was drawn by venopuncture into plastic tubes that contained 1/10 volume 3.8% citrate and platelet-rich plasma (PRP) was prepared in accordance with known techniques (Soslau, G. and J. Giles. 1982. Thrombos. Res. 26:443-455). Blood samples were obtained from healthy donors who were medication free. Washed platelets were prepared from the PRP by known methods (Basheer, A.R. et al. 1995. Biochim. Biophys. Acta 1250:97-109). Briefly, PRP was diluted with 3 volumes of 100 nM citrate buffer (pH 6.0) plus 1 to 2 volumes of hepes Tyrode's buffer (pH 7.4), final volume of 50 ml, and resuspended in hepes Tyrode's bluffer with 1 mg/ml dextrose plus 1 mg/ml bovine serum albumin at 2 x 10⁶/µl to 3 x 10⁶/µl (a 10X normal concentration).

Platelet aggregations were performed on a dual-channel Chronolog lumi aggregometer (Chronolog Corp., Havertown, PA) by known methods (Soslau, G. et al. 1988. Biochem. Biophys. Res. Commun. 15:909-916). Aggregations were conducted with 480 µl washed platelets or a 50 µl sample of the concentrated platelets added to 430 µl hepes Tyrode's buffer with a final platelet count of 2 x 10⁵/µl to 3 x 10⁵/µl. Agonists and antagonists were added at various concentrations.

## Claims

1. The use of histone compound and a pharmaceutically acceptable vehicle in the manufacture of an agent for preventing platelet aggregation.

2. The use af a histone compound and a pharmaceutically acceptable vehicle in the manufacture of an agent for treating cardiovascular disorders related to thrombotic events.

3. The use according to claim 1 or claim 2 wherein the histone compound comprises H1, H2A, H2B, H3 or H4.

4. The use according to claim 3 wherein the H1 histone is a histone subtype H1.0, H1.1, H1.2, H1.3, H1.4 or H1.5.

5. The use according to claim 1 or claim 2 wherein the histone compound is recombinantly produced.

6. The use according to claim 1 or claim 2 wherein the histone compound is human.

7. The use according to claim 2 wherein the histone compound is administered prophylactically.

8. A histone compound and a pharmaceutically acceptable vehicle for use in preventing platelet aggregation.

9. A histone compound and a pharmaceutically acceptable vehicle for use in preventing thrombotic events.

10. A histone compound according to claim 9 wherein the histone compound comprises H1, H2A, H2B, H3 or H4.

11. A histone compound according to claim 10 wherein the H1 histone is as histone subtype H1.0, H1.1, H1.2, H1.3, H1.4 or H1.5.

12. A histone compound according to claim 8 or a claim 9wherein the histone compound is recombinantly produced.

13. A histone compound according to claim 8 or a claim 9 wherein the histone compound is human.

14. A histone compound according to claim 9 wherein the histone compound is administered prophylactically

## Patentansprüche

1. Verwendung einer Histonverbindung und eines geeigneten pharmazeutischen Vehikels zur Herstellung eines Präparates zur Verhinderung von Blutplättchenaggregation.

2. Verwendung einer Histonverbindung und einem geeigneten pharmazeutischen Vehikel zur Herstellung eines Präparates zur Behandlung von kardiovaskulären Störungen bei thrombotischen Ereignissen.

3. Verwendung nach Anspruch 1 und 2 wobei die Histonverbindung H1, H2A, H2B, H3 oder H4 enthält.

4. Verwendung nach Anspruch 3 wobei Histon H1 ein Histon-Subtyp H1.0, H1.1, H1.2, H1.3, H1.4 oder H1.5 ist.

5. Verwendung nach Anspruch 1 oder 2 wobei die Histonverbindung rekombinant hergestellt ist.

6. Verwendung nach Anspruch 1 oder 2 wobei es sich um eine humane Histonverbindung handelt.

7. Verwendung nach Anspruch 2 wobei die Histonverbindung prophylaktisch eingesetzt wird.

8. Histonverbindung und eine geeignete pharmazeutische Trägersubstanz zur Verhinderung von Blutplättchenaggregation.

9. Histonverbindung und eine geeignete pharmazeutische Trägersubstanz zur Verwendung zwecks Verhinderung von thrombotischen Ereignissen.

10. Histonverbindung nach Anspruch 9, wobei die Histonverbindung H1, H2A, H2B, H3 oder H4 enthält.

11. Histonverbindung nach Anspruch 10, wobei Histom H1 ein Histon-Sybtyp H1.0, H1.1, H1.2, H1.3, H1.4 oder H1.5 ist.

12. Histonverbindung nach Anspruch 8 oder 9, wobei die Histonverbindung rekombinant hergestellt ist.

13. Histonverbindung nach Anspruch 8 oder 9, wobei es sich um eine humane Histonverbindung handelt.

14. Histonverbindung nach Anspruch 9, wobei die Histonverbindung prophylaktisch eingesetzt wird.

## Revendications

1. Utilisation de composé d'histone et d'un véhicule pharmaceutiquement acceptable dans la fabrication d'un agent destiné à la prévention de l'agrégation plaquettaire.

2. Utilisation de composé d'histone et d'un véhicule pharmaceutiquement acceptable dans la fabrication d'un agent destiné au traitement de troubles cardiovasculaires associés à des événements thrombotiques.

3. Utilisation selon la revendication 1 ou la revendication 2, où le composé d'histone comprend H1, H2A, H2B, H3 ou H4.

4. Utilisation selon la revendication 3, où l'histone H1 est un sous-type d'histone H1.0, H1.1, H1.2, H1.3, H1.4 ou H1.5.

5. Utilisation selon la revendication 1 ou la revendication 2, où le composé d'histone est produit de manière recombinante.

6. Utilisation selon la revendication 1 ou la revendication 2, où le composé d'histone est humain.

7. Utilisation selon la revendication 2, où le composé d'histone est administré de manière prophylactique.

8. Composé d'histone et véhicule pharmaceutiquement acceptable en vue d'une utilisation dans la prévention de l'agrégation plaquettaire.

9. Composé d'histone et véhicule pharmaceutiquement acceptable en vue d'une utilisation dans la prévention d'événements thrombotiques.

10. Composé d'histone selon la revendication 9, où le composé d'histone comprend H1, H2A, H2B, H3 ou H4.

11. Composé d'histone selon la revendication 10, où l'histone H1 est un sous-type d'histone H1.0, H1.1, H1.2, H1.3, H1.4 ou H1.5.

12. Composé d'histone selon la revendication 8 ou la revendication 9, où le composé d'histone est produit de manière recombinante.

13. Composé d'histone selon la revendication 8 ou la revendication 9, où le composé d'histone est humain.

14. Composé d'histone selon la revendication 9, où le composé d'histone est administré de manière prophylactique.
